# EUROPÄISCHE PATENTSCHRIFT

(11) **EP 2 164 545 B1**
(45) Veröffentlichungstag und Bekanntmachung des Hinweises auf die Patenterteilung: **20.03.2019**
(21) Anmeldenummer: 08759289.5
(22) Anmeldetag: 19.06.2008
(51) Int. Cl.: A61M 5/30, A61M 5/20

(54) **EINWEGINJEKTOR MIT MINDESTENS EINEM ZWANGSENTKOPPELBAREN ZUGHAKEN**
DISPOSABLE INJECTOR WITH AT LEAST ONE DRAWER HOOK WHICH CAN BE FORCIBLY DECOUPLED
INSTRUMENT D'INJECTION À USAGE UNIQUE COMPRENANT AU MOINS UN CROCHET DE TRACTION POUVANT ÊTRE DÉSACCOUPLÉ DE FORCE

(30) Priorität: 10.07.2007 DE 102007032463
(43) Veröffentlichungstag der Anmeldung: 24.03.2010
(73) Patentinhaber: LTS LOHMANN Therapie-Systeme AG, 56626 Andernach (DE)
(72) Erfinder: MATUSCH, Rudolf, 35041 Marburg (DE)
(74) Vertreter: Thämer, Wolfgang
(86) Internationale Anmeldenummer: PCT/EP2008/004950
(87) Internationale Veröffentlichungsnummer: WO 2009/006987

(56) Entgegenhaltungen:
- WO-A-2007/073839
- DE-C1- 19 821 933
- GB-A- 2 319 184
- US-A- 3 557 784
- US-A- 3 625 208
- US-A- 4 378 015
- US-A- 4 722 728
- US-A- 5 073 165
- US-A1- 2006 129 089

## Beschreibung

Die Erfindung betrifft einen Einweginjektor mit einem Gehäuse, in dem ein mechanisches Federelement, eine Zylinder-Kolben-Einheit, ein Kolbenbetätigungsstempel und eine Auslöseeinheit angeordnet ist, wobei zumindest ein Teil des Kolbenbetätigungsstempels zwischen dem Federelement und dem Kolben der Zylinder-Kolben-Einheit positioniert ist, wobei das Gehäuse einen Zwischenboden und mindestens einen Zughaken aufweist, wobei der Zwischenboden eine Bohrung hat, in der der Kolbenbetätigungsstempel gradgeführt ist, wobei sich im unteren Bereich des Gehäuses ein Fixierbereich zur Aufnahme der einbaubaren Zylinder-Kolben-Einheit befindet, wobei der Kolbenbetätigungsstempel mittels des Federelements nach unten verschiebbar ist und wobei der Kolbenbetätigungsstempel einen Stempelteller aufweist.

Aus der DE 36 44 984 A1 ist u.a. ein derartiger Injektor bekannt. Er hat einen federvorgespannten Kolbenbetätigungsstempel, dessen rückwärtige Stempelstange an ihrem freien Ende elastische Zughaken aufweist. Die Zughaken halten den Kolbenbetätigungsstempel formschlüssig an einer Kante des Injektorgehäuses fest. Sie haben hierzu nur eine geringe Auflagefläche am Gehäuse. Zum Auslösen des Injektors werden die Zughaken von der sie haltenden Kante geschoben. In der Folge schießt der federvorgespannte Kolbenbetätigungsstempel nach vorn, um eine Injektion durchzuführen.

Die US 3,557,784 ist ein Mehrweg-Injektor bekannt, in dessen Gehäuse pro Anwendung eine neue Zylinder-Kolben-Einheit eingesetzt wird. Dazu wird der Mehrweg-Injektor pro Anwendung fast vollständig demontiert und wieder zusammengebaut. Zum Halten der den Kolbenbetätigungsstempel betätigenden Feder sind am Gehäuse des Injektors zwei einander gegenüber liegende Zughaken über jeweils ein mechanisches Schwenkgelenk gelagert. Die beiden Schwenkgelenke sind an der Außenseite des Gehäuses angeordnet.

Aus der US 2006/0129089 A1 ist ein hochkomplizierter, vielteiliger Mehrweg-Injektor bekannt. Er hat einen auf Zug belasteten Kolbenbetätigungsstempel, der mittels eines mehrere Finger aufweisenden Rings am Gehäuse abgestützt ist. Die Abstützung erfolgt unter Zwischenschaltung anderer, separater Gehäuseteile.

Aus der GB 2 319 184 A ist ein Injektor mit einer federbelasteten, selbstsichernden Auslösevorrichtung bekannt. Beim Eindrücken eines Auslöseelements werden Gehäusezungen nach außen verdrängt und ein Stempel freigegeben. Aufgrund der Geometrie der Gehäusezungen wird der Stempel nur schrittweise freigegeben.

Der vorliegenden Erfindung liegt daher die Problemstellung zugrunde, einen modular aufgebauten Einweginjektor zu entwickeln, der bei geringer Baugröße nur wenige Bauteile aufweist und bei einfacher Handhabung eine sichere Lagerung und Funktion gewährleistet.

Diese Problemstellung wird mit den Merkmalen des Hauptanspruchs gelöst. Dazu hat der Zughaken im Bereich seines freien Endes eine Abstützfläche. Die Abstützfläche liegt an einer im Bereich von Öffnungen einer zumindest bereichsweise umlaufenden, normal zur Mittellinie orientierten Bundfläche des Stempeltellers an. Im Kolbenbetätigungsstempel ist zumindest ein Teil der Auslöseeinheit geführt und gelagert. Im Kolbenbetätigungsstempel ist als Auslöseeinheit ein Spreizgetriebe gelagert, dessen spreizende Teile - bei Betätigung - die Zughaken vom Stempelteller lösen.

Mit der Erfindung wird hier beispielsweise ein nadelfreier Einmalinjektor vorgestellt, dessen Kolbenbetätigungsstempel bei einem Auslösevorgang des Einweginjektors freigegeben wird. Dazu wird zum Vorspannen und Halten des Federenergiespeichers der Kolbenbetätigungsstempel über mindestens einen im Gehäuse integrierten Zughaken formschlüssig gehalten. Zum Auslösen des Injektors werden der oder die Zughaken freigegeben, so dass sich der Kolbenbetätigungsstempel - unter der Wirkung des Federenergiespeichers - zumindest annähernd parallel zur Mittellinie des Einweginjektors bewegen kann.

Die Erfindung ist in Anspruch 1 definiert. Weitere Einzelheiten der Erfindung ergeben sich aus den Unteransprüchen und den nachfolgenden Beschreibungen von einigen schematisch dargestellten Ausführungsbeispielen.
- Figur 1:: Einweginjektor mit zwei zwangsentkoppelbaren Zughaken;
- Figur 2:: wie Figur 1, jedoch entsichert und betätigt;
- Figur 3:: wie Figur 2, jedoch nach dem Medikamentenausstoß;
- Figur 4:: Querschnitt zu Figur 1 im Bereich des Sicherungselementes;
- Figur 5:: Einweginjektor mit zwei in Sperrstellung verformten Zughaken;
- Figur 6:: wie Figur 5, jedoch entsichert und betätigt;
- Figur 7:: Innenansicht einer Gehäusehälfte des Einweginjektors nach Figur 5;
- Figur 8:: Seitenansicht zu Figur 7;
- Figur 9:: Einweginjektor mit zwei in Lösestellung verformten Zughaken;
- Figur 10:: Querschnitt zu Figur 9 im Bereich des Sicherungselementes;
- Figur 11:: Teillängsschnitt durch den Kolbenbetätigungsstempel;
- Figur 12:: Teilansicht des Kolbenbetätigungsstempels (fiktiver Zustand);
- Figur 13:: wie Figur 9, jedoch entsichert und betätigt;
- Figur 14:: wie Figur 13, jedoch nach dem Medikamentenausstoß.

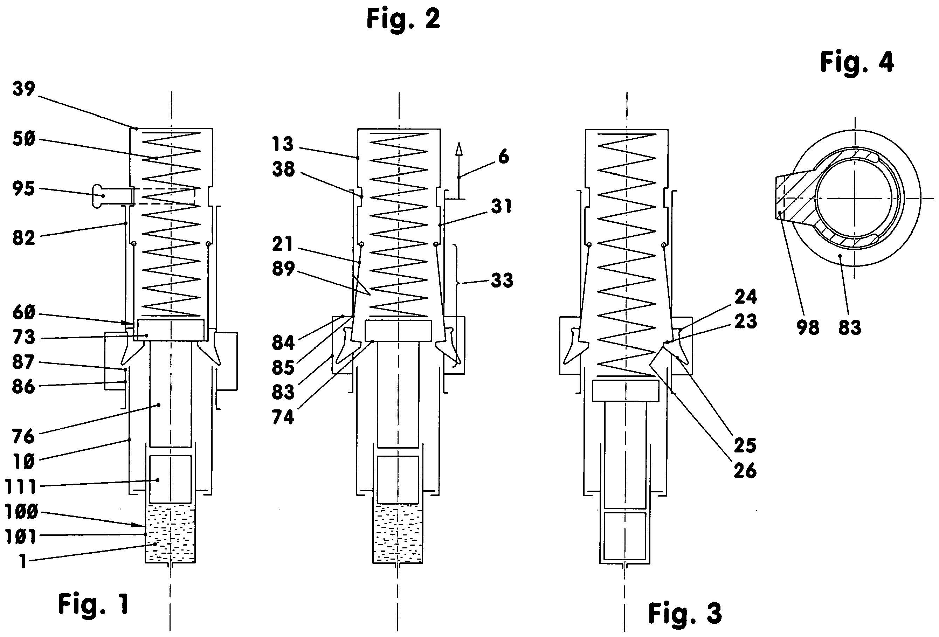

Die Figuren 1 bis 4 zeigen ein vereinfachtes Prinzip eines Einweginjektors mit einem dauergeladenen Federenergiespeicher. Der Einweginjektor besteht aus einem Gehäuse (10), einer z.B. mit einer Injektionslösung befüllten Zylinder-Kolben-Einheit (100), einem Kolbenbetätigungsstempel (60) und einer Schraubendruckfeder (50) als Federenergiespeicher. Zudem sind am Gehäuse (10) ein Auslöseelement (82) und ein Sicherungselement (95) angeordnet.

Das Gehäuse (10) ist ein topfförmiger, unten offener Hohlkörper mit obenliegendem Boden (39). Im mittleren Bereich, dem Mantelbereich (31), hat das Gehäuse (10) z.B. zwei einander gegenüberliegende fensterartige Durchbrüche (33). Am jeweils oberen Rand eines Durchbruches (33) ist jeweils ein Zughaken (21) gelenkig gelagert. Die beiden Zughaken (21) halten den Kolbenbetätigungsstempel (60) an dessen Stempelteller (73) in seiner vorgespannten Lage. Dazu umgreifen die Zughaken (21) mit ihren relativ großen Abstützflächen (23) die untere Stirnseite (74) des Stempeltellers (73). Die Größe der jeweiligen Kontaktfläche zwischen einer Abstützfläche (23) und der entsprechenden Stirnseitenfläche (74) liegt im Bereich von 10 bis 20 mm².

An die Abstützflächen (23) schließt sich zur Mittellinie (5) hin jeweils eine Innenfläche (26) an. Letztere geht hier in eine Keil- oder Konusfläche (25) über. Die Keilfläche (25) kann auch sphärisch gekrümmt sein.

Auf der der Mittellinie (5) abgewandten Seite weist jeder Zughaken (21) eine Anlagefläche (24) auf.

Im hinteren Bereich des Gehäuses (10) befindet sich eine Ringnut (38) zur Aufnahme des Sicherungselements (95). Im unteren Bereich des Gehäuses (10) befinden sich Halteelemente zur Befestigung der Zylinder-Kolben-Einheit.

Die Zylinder-Kolben-Einheit (100) besteht im Ausführungsbeispiel aus einem, mit einer Injektionslösung (1) befüllten, Zylinder (101), in dem ein Kolben (111) in der hinteren Position sitzt. Oberhalb des Kolbens (111) ist im Gehäuse (10) der Kolbenbetätigungsstempel (60) z.B. so angeordnet, dass er den Kolben zwar nicht berührt, jedoch mit seinem unteren Ende im oberen Bereich des Zylinders (101) seitlich geführt wird.

Nach Figur 1 ist das Gehäuse (10) unterhalb der Ringnut (38) vom dem hülsenartigen Auslöseelement (82) umgeben. Das Auslöseelement (82) ist auf der radialen Außenfläche (13) des Gehäuses (10) längsverschiebbar gelagert. Es hat im unteren Bereich auf der Höhe der Hakenenden eine umlaufende Aufweitung (83), vgl. Figur 4. Anstelle dieser Aufweitung (83) können bei einem nichtrotationssymmetrischen Auslöseelement (82) pro Zughaken (21) auch partielle Aufweitungen oder nicht abgedeckte Öffnungen vorhanden sein.

Die Aufweitung (83) ist im Bezug auf das Gehäuse (10) genau so positioniert und dimensioniert, dass sie die beim Auslösevorgang zurückweichenden, nach außen gedrängten Zughaken (21) aufnehmen kann. Die Innenkontur der Aufweitung (83) ist ein Kanal mit einer Rücksprungflanke (84), die hier eine zur Mittellinie (5) des Injektors normale Ebene darstellt. Der Übergang zwischen der beispielsweise zylindrischen Innenwandung des Auslöselementes (82) und der Rücksprungflanke (84) ist z.B. als scharfkantige Kante (85) ausgebildet.

Der Rücksprungflanke (84) gegenüber liegt eine Auslöseflanke (86). Letztere hat eine Auslösekante (87), die nach den Figuren 1 bis 3 die Zughakenenden zumindest bereichsweise umgreift. In Figur 1 berühren die Auslösekanten (87) die Zughaken (21) nicht. Die die Auslösekante (87) tragende Wandung des hülsenartigen Auslöseelements (82) liegt hier ebenfalls gleitend an der Außenwandung (13) des Gehäuses (10) an.

Nach Figur 1 liegen die Zughaken (21) mit ihren außen liegenden Anlageflächen (24) an der Innenwandung (89) des Auslöseelements (82) sichernd an. Bei den Figuren 2 und 3 kontaktieren die Auslösekanten (87) die Keil- bzw. Konusflanken (25) der Zughaken (21).

Der im Gehäuse (10) angeordnete Kolbenbetätigungsstempel (60) ist hier in zwei Bereiche aufgeteilt. Der untere Bereich ist der Kolbenschieber (76). Sein Durchmesser ist etwas kleiner als der Innendurchmesser des Zylinders (101) der Zylinder-Kolben-Einheit (100). Die untere Stirnfläche des Kolbenschiebers (76) wirkt direkt auf den Kolben (111).

Der obere Bereich, der Stempelteller (73), ist eine flache, zumindest bereichsweise zylindrische Scheibe, deren Außendurchmesser einige Zehntel Millimeter kleiner ist als der Innendurchmesser des Gehäuses (10) im Mantelbereich (31).

Der Kolbenschieber (76) kann selbstverständlich auch als separates, vom Stempelteller (73) getrenntes, Bauteil ausgeführt sein. Hierzu ist er dann an der Innenwandung des Gehäuses (10) geführt.

Zwischen dem Stempelteller (73) und dem obenliegenden Boden (39) des Gehäuses (10) sitzt vorgespannt die Schraubendruckfeder (50).

Das Auslöseelement (82) liegt nach Figur 1 an einem Sicherungselement (95) an. Letzteres sitzt in der Ringnut (38). Das Sicherungselement (95) ist beispielsweise ein elastischer omegaförmiger Bügel, der die Ringnut (38) des Gehäuses (10) auf einem Winkel von ca. 120 Winkelgraden federnd umschließt. Nach den Figuren 1 und 4 hat er auf der linken Seite einen Griff (98), über den er - zum Entsichern - seitlich abgezogen werden kann.

Zum Betätigen des Einweginjektors wird - nach dem Entfernen einer Düsenversiegelung - zunächst das Sicherungselement (95) seitlich abgezogen. Dann wird das Auslöseelement (82) mit der zur Faust geformten Hand umgriffen und der Einweginjektor auf der Injektionsstelle positioniert. Hierbei befindet sich der Daumen am hinteren Ende des Einweginjektors. Er liegt auf dem Boden (39) des Gehäuses (10) auf. Zum unmittelbaren Auslösen wird der Daumen, der den Einweginjektor tragenden Hand, gegen den Boden (39) gepresst. Bei diesem Vorgang gleitet das Auslöseelement (82) auf der Außenwandung (13) des Gehäuses (10) nach hinten, also weg von der Injektionsstelle. Die Anlageflächen (24) der Zughaken (21) rutschen über die Kante (85) und sind somit entsichert. Zeitgleich oder kurz danach kontaktiert die Auslösekante (87) die Keilfläche (25). Durch die Aufwärtsbewegung (6) des Auslöseelements (82) schiebt die Auslösekante (87) über die Keilfläche (25) den jeweiligen Zughaken (21) nahezu radial nach außen, vgl. Figur 2. Die Auslösekante (87) des Auslöseelements (82) und die Keilfläche (25) des einzelnen Zughakens (21) bilden zusammen jeweils ein zwangsläufiges Schiebekeilgetriebe.

Das Zughakenende weicht in die Aufweitung (83) aus und gibt dabei den Stempelteller (73) frei. Nun kann der Kolbenbetätigungsstempel (60) ungehindert nach unten schnellen, vgl. Figur 3. Der Zylinder (101) wird entleert.

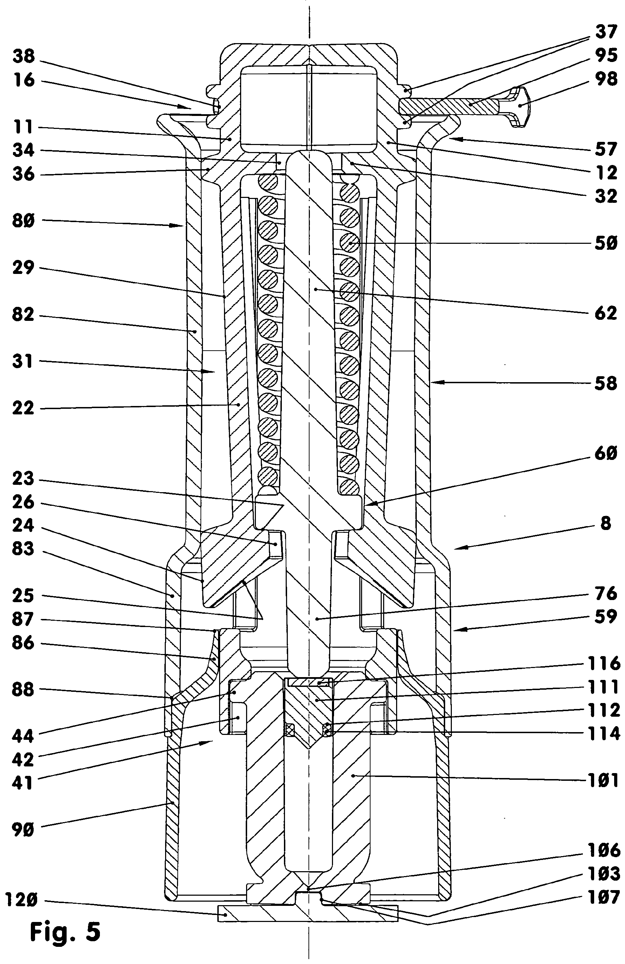

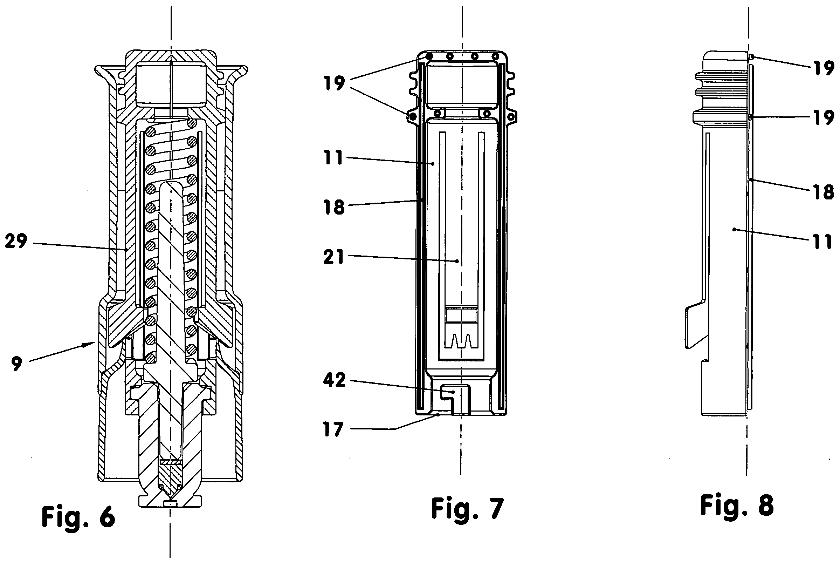

Die Figuren 5 bis 8 zeigen eine Ausführungsform des in den Figuren 1 bis 4 beschriebenen Prinzips. Hier ist das tragende Bauteil ein zweiteiliges Gehäuse (10). Es besteht aus zwei beispielsweise identische Halbschalen (11, 12), deren Montagefuge in einer auf der Mittellinie (5) gelegenen Ebene liegt. Beide Halbschalen sind z.B. aus einem glasfaserverstärkten Polyamid durch Spritzgießen gefertigt. In der Montagezone hat jede Halbschale mindestens einen erhabenen Steg (18) und z.B. vier Zapfen (19), vgl. Figur 7 und 8. Die Zapfen (19) und der Steg (18) der einen Halbschale (11) greift nach dem Verkleben beider Teile in entsprechende Aussparungen der anderen Halbschale (12).

Das montierte Gehäuse (10) hat eine weitgehend rohrförmige Gestalt und ist in drei Funktionsbereiche (16, 31, 41) aufgeteilt. Nach den Figuren 5 und 6 ist der obere Bereich der Auslösebereich (16). An ihn schließt sich der Mantelbereich (31) an. Zwischen beiden Bereichen ist ein Zwischenboden (32) angeordnet. Der Zwischenboden (32) hat eine zentrale Ausnehmung (34).

Im Auslösebereich (16) befindet sich oberhalb des Zwischenbodens (32) ein geschlossener Boden (39). Zwischen beiden Böden (32, 39) weist die Außenwandung des Gehäuses (10) zwei umlaufende Stege (37) auf. Die zwischen den Stegen (37) gelegene Ringnut (38) dient der Aufnahme eines Sicherungselements (95), vgl. Figur 5.

Im Mantelbereich (31) einer jeden Halbschale (11, 12) befindet sich ein angeformter Zughaken (21), vgl. auch Figuren 7 und 8. Die Anformstelle für die Zughaken (21) liegt knapp unterhalb des Zwischenbodens (32). Dort hat die Auswandung des Gehäuses (10) einen umlaufenden Stützsteg (36).

Zur Ausbildung des jeweiligen Zughakens (21) befindet sich im Mantelabschnitt (31) ein schmaler, zumindest annähernd u-förmiger, Spalt. Der obere Bereich des Zughakens (21), das entspricht ca. 80% der Zughakenlänge, entspricht bezüglich der Wandstärke und der Krümmung der Wandung des Gehäuses (10). Dieser Bereich hat die Funktion eines federelastischen Biegebalkens (22). Er hat einen sichelförmigen Querschnitt.

Ggf. kann ein Teil dieses Biegebalken (22) auch mit einem rechteckigen Querschnitt ausgestattet sein, um bei der Nutzung auftretende Biegespannungen im Biegebalkenrandbereich zu reduzieren. In den Figuren 7 und 8 ist der Zughaken (21) im unverformten Zustand dargestellt.

Das hier untere freie Ende des einzelnen Zughakens (21) ist als Haken mit zumindest drei funktionalen Flächen (23, 24, 25) ausgebildet. Eine für einen Haken umgriffartige Fläche bildet die Abstützfläche (23), auf der bei gespanntem Einweginjektor der Kolbenbetätigungsstempel (60) über seine untere Stirnfläche (74) aufliegt. Ggf. haben die Zughaken (21) zumindest im Bereich der Abstützflächen (23) eine keramische Panzerung.

Die nach unten orienierte Fläche des Zughakens (21) ist die sog. Keilfläche (25). Die Keilflächen (25) der beiden verformten Zughaken (21), vgl. Figur 5 sind Abschnitte eines Kegelmantels, dessen Spitze auf der Mittellinie (5) im Bereich des Stempeltellers (73) liegt. Der gedachte Kegelmantel hat hier z.B. einen Kegelwinkel von 100 Winkelgraden.

An die Keilfläche (25) schließt sich die radial nach außen ausgerichtete Anlagefläche (24) an. Die Anlagefläche (24) steht nach den Figuren 6 und 8 um z.B. die 1,6-fache Biegebalkenwandstärke radial nach außen über die Außenwandung des Gehäuses (10) über.

Nach Figur 7 weist das untere Ende des Zughakens (21) z.B. zwei Kerben auf.

Unterhalb des Mantelabschnitts (31) befindet sich der Fixierbereich (41) zur Aufnahme der einbaubaren Zylinder-Kolben-Einheit (100). Der Fixierbereich (41) ist Teil eines Bajonettverschlusses. Dazu sind an seiner Innenwandung zwei oder mehrere winkelförmige Kanäle (42) angeordnet, vgl. Figur 7. Die Kanäle (42) führen von der unteren Gehäusestirnseite (17) aus vertikal nach oben und gehen nach wenigen Millimetern Länge jeweils in einen kurzen horizontalen Kanalabschnitt über.

Im Fixierbereich (41) ist der Zylinder (101) über z.B. zwei oder mehrere Bajonettzapfen (44) eingesetzt und fixiert, vgl. Figur 5. Ggf. befindet sich im horizontalen Kanalabschnitt oder an zumindest einem Teil der Bajonettzapfen (44) ein oder mehrere Rastelemente, die ein Lösen des Bajonettverschlusses - also ein Entfernen des Zylinders (101) - verhindern.

Der Zylinder (101) ist z.B. ein dickwandiger Topf. In der beispielsweise zylindrischen Bohrung des Zylinders (101) sitzt der stangenlose Kolben (111). Der Kolben (111) hat an seiner vorderen, zumindest annähernd kegelig gestalteten Stirnfläche eine axiale Ringnut (112) zur Aufnahme eines Dichtringes (114) oder einer dauerelastischen Dichtmasse. In der rückseitigen Stirnfläche des Kolbens (111) ist eine z.B. zylindrische Metallplatte (116) eingelassen.

Im Zentrum der Bohrung des Zylinders (101), dessen Zylinderboden der Kontur der vorderen Kolbenstirnseite zumindest annähernd angepasst ist, befindet sich eine kurze zylindrische, düsenartige Bohrung (106). Ihr Durchmesser beträgt ca. 0,1 bis
0,5 Millimeter. Diese Bohrung (106) ist ein- bis fünfmal so lang wie ihr Durchmesser. Sie endet in einer zylindrischen Ausnehmung (107) der bodenseitigen, äußeren Stirnfläche (103) des Zylinders (101).

Zwischen dem Kolben (111) und dem Zwischenboden (32) ist der Federenergiespeicher (50) bzw. die Antriebseinheit des Einweginjektors angeordnet. Der Federenergiespeicher (50) ist eine Schraubendruckfeder, die auf dem Kolbenbetätigungsstempel (60) mit dem Stempelteller (73) angeordnet ist. Mittels des Stempeltellers (73) stützt sich der federkraftbelastete Kolbenbetätigungsstempel (60) an den Zughaken (21) des Gehäuses (10) ab.

Der Kolbenbetätigungsstempel (60) hat oberhalb des Stempeltellers (73) einen Führungszapfen (62). Letzterer führt die Schraubendruckfeder (50). Unterhalb des Stempeltellers (73) befindet sich zentral in der Verlängerung des Führungszapfens (62) ein Kolbenschieber (76), der bei einer Betätigung des Einmalinjektors auf den Kolben (111) wirkt.

Das teilweise das Gehäuse (10) und die Zylinder-Kolben-Einheit umgebende Auslöseelement (82) ist hier ebenfalls eine Auslösehülse. Die rotationssymmetrische, z.B. aus ABS gefertigte, Auslösehülse (82) hat drei Bereiche, einen Einführungsbereich (57), einen Mittenbereich (58) und einen Aufweitungsbereich (59). Der Mittenbereich (58) kann ein zumindest annähernd zylindrisches Rohr sein. Im Ausführungsbeispiel besteht er aus zwei nahezu gleichlangen kegelstumpfmantelartigen Rohrabschnitten, die sich von ihrer geometrischen Mitte aus nach oben und unten, vgl. Figur 5 aufweiten. Der jeweilige Kegelwinkel beträgt z.B. 2 Winkelgrade.

In Figur 5 schließt sich nach oben hin an den Mittenbereich (58) der sich trichterförmig aufweitende Einführbereich (57) an. Letzterer hat einen Kegelwinkel von 100 bis 110 Winkelgraden. Der maximale Durchmesser des Einführbereiches (57) ist größer als der maximale Außendurchmesser des montierten Gehäuses (10) im Bereich der unteren Enden der Zughaken (21).

An den Mittenbereich grenzt unten der Aufweitungsbereich (59) an. Er ist ebenfalls ein geringfügig kegelmantelstumpfförmiger Rohrabschnitt, dessen Querschnitt sich kontinuierlich nach unten hin vergrößert. Sein Kegelwinkel beträgt ca. 2 Winkelgrade. Zwischen dem Mittenbereich (58) und dem Aufweitungsbereich (59) befindet sich eine Übergangszone. Da an dieser Stelle der Aufweitungsbereich (59) einen Durchmesser hat, der um ca. zwei Auslösehülsenwandstärken größer ist als der Außendurchmesser des angrenzenden Mittenbereichs (58), bildet die Übergangszone dort einen Absatz.

Der untere Rand des Aufweitungsbereichs (83) weist eine Eindrehung (88) auf. In dieser Eindrehung (88) sitzt eingeklebt oder verschweißt ein z.B. aus Polycarbonat (PC) oder Polymethylmethacrylat (PMMA) gefertigtes Splitterschutzrohr (90). Das transparente Splitterschutzrohr (90), das die Zylinder-Kolben-Einheit (100) großteils umgibt, verjüngt sich zum Gehäuse (10) hin zumindest annähernd trichterartig so weit, dass seine Innenwandung die Außenwandung des Gehäuses (10) mit Spiel kontaktiert. Die in diesem Bereich liegende obere Vorderkante des Splitterschutzes (90) ist die hier kreisringförmig umlaufende Auslösekante (87), die sich an die Auslöseflanke (86) anschließt. Die Auslöseflanke (86) hat unterhalb der Auslösekante (87) einen Kegelwinkel, der unter 60 Winkelgraden liegt.

Die Figur 6 zeigt den Einweginjektor ohne Sicherungselement (95), also entsichert, mit hochgezogenem Auslöseelement (82).

Mit dem Hochziehen des Auslöseelements (82), das auch als ein Niederdrücken des Gehäuses (10) bezeichnet werden kann, rutschen die Anlageflächen (24), vgl. Figur 5, entlang der Rücksprungflanke (84) nach außen. Das nach außen Biegen der Zughaken (21) wird erzwungen, sobald die hakenseitigen Keilflächen (25) an der Kante (85), also dem oberen Rand des Splitterschutzes (90) zwangsweise entlang gleiten. Die Zughaken (21), die nun nicht mehr verformt sind, geben hierbei den Kolbenbetätigungsstempel frei, so dass der Kolben (111) in den Zylinder (101) ruckartig eingeschoben wird.

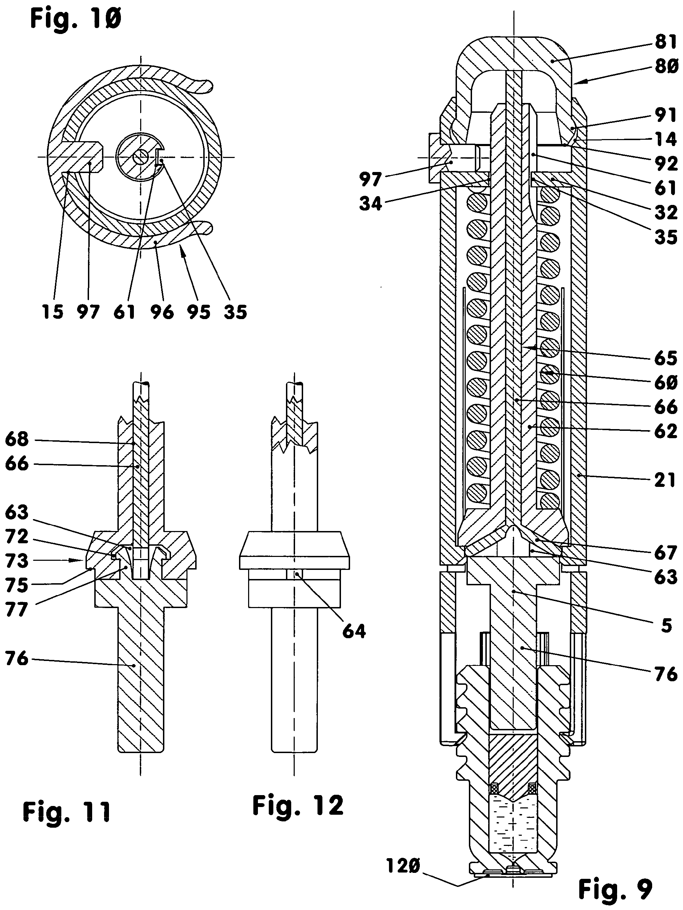

Die Figuren 11 bis 14 stellen eine weitere Zwangsentriegelung für einen Zughaken-Injektor dar. Hier werden die Zughaken (21) vom Kolbenbetätigungsstempel (60) mittels eines in der Auslöseeinheit (80) integrierten Spreizgetriebes nach außen geschoben.

Hierzu hat der Injektor einen besonderen Kolbenbetätigungsstempel (60). Oberhalb des Kolbenschiebers (76) ist er zumindest bereichsweise hohl ausgeführt. Er hat eine zentrale Bohrung (62), deren Mittellinie z.B. deckungsgleich mit der Mittellinie (5) zusammenfällt. Die Bohrung (62) endet im Stempelteller (73) in einer Ausnehmung (63), die hier zwei einander gegenüberliegende, z.B. radiale Öffnungen (64) hat, vgl. Figur 12. Im Bereich des Stempeltellers (73) ist der Kolbenbetätigungsstempel (60) z.B. aus Fertigungsgründen geteilt. Der Kolbenschieber (76) hat zwei Widerhaken (77), mit denen er in entsprechenden Aussparungen (72) des Stempeltellers (73) z.B. unlösbar verrastet, vgl. Figur 11.

Der Stempelteller (73) hat im Bereich der Öffnungen (64) eine zumindest bereichsweise umlaufende Bundfläche (75), die normal zur Mittellinie (5) orientiert ist. Auf dieser Bundfläche (75) liegen die Zughaken (21) mit ihren Abstützflächen (23) an.

In der Bohrung (68) und der Ausnehmung (63) ist ein gegabelter Druckstab (65) eingesteckt. Der Druckstab (65) hat hier einen zylindrischen Schaft (66), der in der Bohrung (68) mit Spiel geführt ist. Am unteren Ende des Schaftes (66) sind zwei Pendelstangen (67) gelenkig angeformt. Die Pendelstangen (67) haben z.B. einen rechteckigen Querschnitt. Die Gelenke zwischen dem Schaft (66) und den Pendelstangen (67) sind beispielsweise Filmgelenke. Ggf. sind nur die Pendelstangen (67) miteinander verbunden und der Schaft bildet ein separates Bauteil.

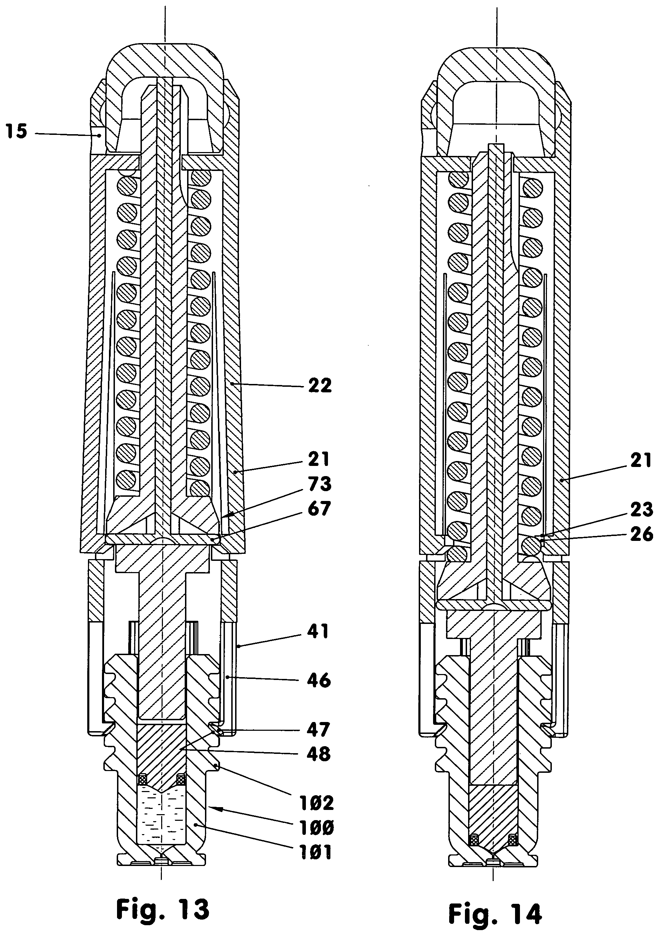

Nach Figur 9 bilden die Pendelstangen (67) des Druckstabs (65) im unbetätigten Injektor die gleichlangen Schenkel eines gleichschenkeligen Dreiecks. Die freien Enden der Pendelstangen (67) liegen an den jeweiligen Innenflächen (26), vgl. Figur 14, der Zughaken (21) an.

Das obere, freie Ende des Druckstabes (65) ragt oben aus der Bohrung (68) heraus. An der Stirnfläche des Schaftes (66) liegt ein Druckknopf (81) mit seinem Boden an.

Der Druckknopf (81) hat prinzipiell die Form einer Büchse, die aus einem Boden und einer Schürze besteht. Er hat außen im unteren Bereich mindestens zwei radial vorstehende Nocken (91). Die Nocken (91) greifen bei dem montierten und gesicherten Druckknopf (81) in Vertiefungen (14) des Gehäuses (10) ein, vgl. Figur 9.

Die Schürze hat einen unteren, z.B. ebenen Rand (92), der beim Betätigen des Druckknopfes (81) als Anschlag gegenüber dem Zwischenboden (32) des Gehäuses (10) dient. Im unbetätigten und gesicherten Zustand liegt der Rand (92) auf einem Sperrbolzen (97) eines Sicherungselements (95) auf.

Das Sicherungselement (95) besteht aus dem Sperrbolzen (97) und einem diesen tragenden offenen, federnden Ring in Form einer Omegafeder (96), vgl. Figur 10. Am gesicherten Injektor sitzt die Omegafeder (96) auf der Außenwandung des Gehäuses (10). Sie umgibt die Außenwandung auf ca. 240 Winkelgraden. Der Sperrbolzen (97) steckt hierbei in einer Bohrung (15) des Gehäuses (10). Er ragt wenige Millimeter in das Gehäuseinnere hinein, vgl. Figur 9. Ggf. ist mindestens eines der freien Enden der Omegafeder (96) mit einer Papier- oder Folienbanderole plombiert.

Zur Ausrichtung des Kolbenbetätigungsstempels (60) gegenüber dem Gehäuse (10) ragt der Zwischenboden (32) mit einer Verdrehsicherung (35) in eine Führungsnut (61) des Kolbenbetätigungsstempels (60) hinein. Zur Verdrehsicherung kann der Querschnitt des Kolbenbetätigungsstempels (60) und die Bohrung (34) auch rechteckig, oval, elliptisch oder dergleichen ausgeführt sein.

Im unteren Bereich des Gehäuses (10), vgl. Figur 13, befindet sich der Fixierbereich (41) zur Aufnahme der einbaubaren Zylinder-Kolben-Einheit (100). Der Fixierbereich (41) umfasst z.B. sechs Federhaken (46), die jeweils in einer nach innen gerichteten Hakenspitze (47) enden. Die Hakenspitzen (47) haben zur unteren Gehäusestirnseite hin eine sich über die gesamte Hakenstärke erstreckende Anfasung (48). Die Länge und die Federrate der Federhaken (46) ist so dimensioniert, dass die für die Funktion des Einweginjektors erforderlichen Einbauten (50, 100) ohne plastische Verformung der Federhaken (46) eingebaut werden können.

Der Zylinder (101) der Zylinder-Kolben-Einheit (100) ist hier ein dickwandiger Topf, dessen ggf. zylindrische Außenwandung beispielsweise fünf umlaufende Rastrippen (102) trägt. Die Summe der Rastrippen (102) hat im Querschnitt z.B. ein Sägezahnprofil, wobei die Teilung zwischen den zahnartigen Rastrippen (102) äquidistant ist. Der maximale Durchmesser der Rastrippen (102) ist geringfügig kleiner als der Innendurchmesser des Gehäuses (10) im Fixierbereich (41). Der Durchmesser der zwischen benachbarten Rastrippen (102) liegenden Bereiche entspricht dem minimalen Durchmesser des Gehäuses (10) im Bereich der Hakenspitzen (47).

Zum Betätigen des Injektors wird nach dem seitlichen Herausziehen des Sicherungselements (95) und dem Abziehen der Schutzfolie (120), vgl. Figur 9, der Druckknopf (81) nach unten gedrückt. Hierbei gleiten die Pendelstangen (67) - geschoben vom abwärts bewegten Schaft (66) - in eine Strecklage, vgl. Figur 13. Die freien Enden der Pendelstangen (67) drücken die Zughaken (21) elastisch verformend nach außen, so dass sie den Stempelteller (73) nicht mehr hintergreifen.

Das Federelement (50) schiebt nun den Kolbenbetätigungsstempel (60) nach unten, vgl. Figur 14. Mit der Abgabe des Medikaments über die Zylinder-Kolben-Einheit (100) ist der Injektionsvorgang beendet.

Bei Injektoren, bei denen - wie in der zuletzt beschriebenen Variante - der Kolbenbetätigungsstempel (60) zumindest in der Bohrung (34) des Bodens oder Zwischenbodens (32) geradgeführt ist und der Kolbenbetätigungsstempel (60) eine ausreichende Biegefestigkeit aufweist, kann anstatt zwei oder mehrerer Zughaken (21) auch nur ein einziger Zughaken (21) verwendet werden.

Bei den in den Figuren dargestellten Varianten ist die einzelne Kontaktzone zwischen dem Zughaken (21) und dem Stempelteller (73), als Flächen (23) und (74, 75) ausgeführt, die gleitfähig einander kontaktieren. In einer besonderen Ausgestaltung kann in jeder Fläche (23) der einzelnen Zughaken (21) eine Walze gelagert werden, die bei einer Betätigung des Injektors an der Fläche (74, 75) des Stempeltellers wälzgelagert, also reibungsarm, abrollt.

Mit Ausnahme des Federelements (50) und der ggf. vorhandenen Kolbenplatte (116) sind alle Teile der zuvor beschriebenen Einweginjektoren aus Kunststoffen oder kunststoff- bzw. gummiähnlichen Werkstoffen gefertigt.

### Bezugszeichenliste:

- 1: Injektionslösung; Medikament
- 5: Mittellinie des Einweginjektors
- 6: Auslösebewegungsrichtung von (82), Aufwärtsbewegung
- 8: Sperrstellung
- 9: Lösestellung

- 10: Gehäuse, einteilig
- 11: erste Halbschale
- 12: zweite Halbschale
- 13: Außenfläche, zylindrisch
- 14: Vertiefungen, Ringnut
- 15: Bohrung
- 16: Auslösebereich, oben
- 17: untere Gehäusestirnseite
- 18: Steg
- 19: Zapfen

- 21: Zughaken
- 22: Biegebalken
- 23: Abstützfläche
- 24: Anlagefläche
- 25: Keilfläche, Konusfläche
- 26: Innenfläche
- 29: Rückenfläche

- 31: Mantelbereich
- 32: Zwischenboden
- 33: Durchbrüche
- 34: Bohrung
- 35: Verdrehsicherung
- 36: Stützsteg
- 37: Stege, außen umlaufend
- 38: Ringnut für (95)
- 39: Boden

- 41: Fixierbereich für die Zylinder-Kolben-Einheit
- 42: Kanäle, winkelförmig
- 44: Bajonettzapfen
- 46: Federhaken
- 47: Hakenspitze
- 48: Anfasung

- 50: Federelement, Schraubendruckfeder, Federenergiespeicher

- 57: Einführbereich
- 58: Mittenbereich
- 59: Aufweitungsbereich

- 60: Kolbenbetätigungsstempel
- 61: Führungsnut
- 62: Führungszapfen
- 63: Ausnehmung
- 64: Öffnungen, radial
- 65: Druckstab, gegabelt
- 66: Schaft
- 67: Pendelstangen
- 68: Bohrung

- 72: Aussparungen in (73)
- 73: Stempelteller
- 74: Stirnseite, unten; Stirnseitenfläche
- 75: Bundfläche, Stirnseitenfläche
- 76: Kolbenschieber
- 77: Widerhaken an (76)

- 80: Auslöseeinheit
- 81: Druckknopf
- 82: Auslöseelement
- 83: Aufweitung
- 84: Rücksprungflanke
- 85: Kante, scharfkantig
- 86: Auslöseflanke
- 87: Auslösekante
- 88: Eindrehung, Gehäusebund
- 89: Innenwandung
- 90: Splitterschutzrohr
- 91: Nocken
- 92: Rand, unten

- 95: Sicherungselement
- 96: Omegafeder
- 97: Sperrzapfen, Sperrbolzen
- 98: Griff

- 100: Zylinder-Kolben-Einheit
- 101: Zylinder
- 102: Rastrippen, außen; Außenrillung
- 103: Stirnfläche
- 106: Bohrung, Düse
- 107: Ausnehmung in der Stirnfläche
- 111: Kolben
- 112: Ringnut
- 114: Dichtring, Dichtung
- 116: Metallplatte, magnetisch oder magnetisierbar

- 120: Schutzfolie, Klebeversiegelung

## Patentansprüche

1. Einweginjektor mit einem Gehäuse (10), in dem ein mechanisches Federelement (50), eine Zylinder-Kolben-Einheit (100), ein Kolbenbetätigungsstempel (60) und eine Auslöseeinheit (80) angeordnet ist, wobei zumindest ein Teil des Kolbenbetätigungsstempels (60) zwischen dem Federelement (50) und dem Kolben (111) der Zylinder-Kolben-Einheit (100) positioniert ist, wobei das Gehäuse (10) einen Zwischenboden (32) und mindestens einen Zughaken (21) aufweist, wobei der Zwischenboden (32) eine Bohrung (34) hat, in der der Kolbenbetätigungsstempel (60) gradgeführt ist, wobei sich im unteren Bereich des Gehäuses (10) ein Fixierbereich (41) zur Aufnahme der einbaubaren Zylinder-Kolben-Einheit (100) befindet, wobei der Kolbenbetätigungsstempel (60) mittels des Federelements (50) nach unten verschiebbar ist und wobei der Kolbenbetätigungsstempel (60) einen Stempelteller (75) aufweist, **dadurch gekennzeichnet,**
- **dass** der Zughaken (21) im Bereich seines freien Endes eine Abstützfläche (23) hat,
- **dass** die Abstützfläche (23) an einer im Bereich von Öffnungen (64) einer zumindest bereichsweise umlaufenden, normal zur Mittellinie (5) orientierten Bundfläche (75) des Stempeltellers (75) anliegt,
- **dass** im Kolbenbetätigungsstempel (60) zumindest ein Teil (65 - 67) der Auslöseeinheit (80) geführt und gelagert ist und
- **dass** im Kolbenbetätigungsstempel (60) als Auslöseeinheit ein Spreizgetriebe (65 - 67, 73) gelagert ist, dessen spreizende Teile - bei Betätigung - die Zughaken (21) vom Stempelteller (73) lösen.

2. Einweginjektor gemäß Anspruch 1, **dadurch gekennzeichnet, dass** der Kolbenbetätigungsstempel (60) mehrteilig ausgebildet ist.

3. Einweginjektor gemäß Anspruch 1, **dadurch gekennzeichnet, dass** der Kolbenbetätigungsstempel (60) verdrehsicher im Gehäuse (10) gelagert ist.

## Claims

1. A single-use injector comprising a housing (10), in which a mechanical spring element (50), a cylinder-piston unit (100), a piston actuation ram (60), and a triggering unit (80) are arranged, wherein at least part of the piston actuation ram (60) is positioned between the spring element (50) and the piston (111) of the cylinder-piston unit (100), wherein the housing (10) has a false floor (32) and at least one draw hook (21), wherein the false floor (32) has a bore (34), in which the piston actuation ram (60) is guided straight, wherein a fixing region (41) for receiving the cylinder-piston unit (100), which can be installed, is located in the lower region of the housing (10), wherein the piston actuation ram (60) is displaceable downwards by means of the spring element (50), and wherein the piston actuation ram (60) has a ram plate (73), **characterised in that**
- the draw hook (21), in the region of its free end, has a support face (23),
- the support face (23) rests against a collar face (75) of the ram plate (73), said collar face in the region of openings (64) being arranged peripherally at least in some regions and oriented normal to the centre line (5),
- at least part (65-67) of the triggering unit (80) is guided and mounted in the piston actuation ram (60), and
- an expandable gear (65-67, 73) is mounted in the piston actuation ram (60) as triggering unit, the expanding parts of said gear - in the event of actuation - releasing the draw hooks (21) from the ram plate (73).

2. The single-use injector according to claim 1, **characterised in that** the piston actuation ram (60) is formed in a number of parts.

3. The single-use injector according to claim 1, **characterised in that** the piston actuation ram (60) is mounted in the housing (10) in a manner secured against rotation.

## Revendications

1. Injecteur à usage unique comprenant un boîtier (10) dans lequel sont disposés un élément mécanique à ressort mécanique (50), une unité cylindre-piston (100), un poinçon d'actionnement de piston (60) et une unité de déclenchement (80), au moins un élément du poinçon d'actionnement de piston (60) étant positionné entre l'élément à ressort (50) et le piston (111) de l'unité cylindre-piston (100), le boîtier (10) comportant un fond intermédiaire (32) et au moins un crochet de traction (21), le fond intermédiaire (32) présentant un perçage (34) dans lequel le poinçon d'actionnement du piston (60) est guidé d'une manière rectiligne, une zone de fixation (41) destinée à recevoir l'unité cylindre-piston incorporable (100) se trouvant dans la partie inférieure du boîtier (10), le poinçon d'actionnement du piston (60) pouvant être déplacé vers le bas au moyen de l'élément à ressort (50) et le poinçon d'actionnement de piston (60) présentant une plaque d'appui (73),
**caractérisé en ce**
- **que** la tige de traction (21) présente une surface d'appui (23) dans la zone de son extrémité libre,
- **que** la surface d'appui (23) s'applique contre une surface de collet (75) de la plaque d'appui (73), circulaire au moins par endroit dans une zone d'ouvertures (64) et orientée perpendiculairement par rapport à la ligne médiane (5),
- **qu'**au moins un élément (65 - 67) de l'unité de déclenchement (80) est guidé et logé dans le poinçon d'actionnement du piston (60) et
- **qu'**un mécanisme d'expansion (65 - 67, 73) dont les éléments à expansion libèrent les crochets de traction (21) de la plaque d'appui (73) lors de l'actionnement, est logé comme unité de déclenchement dans le poinçon d'actionnement du piston (60).

2. Injecteur à usage unique selon la revendication 1, **caractérisé en ce que** le poinçon d'actionnement de piston (60) est réalisé en plusieurs parties.

3. Injecteur à usage unique selon la revendication 1, **caractérisé en ce que** le poinçon d'actionnement du piston (60) est monté de manière solidaire en rotation dans le boîtier (10).
